# EUROPEAN PATENT SPECIFICATION

(11) **EP 4 472 584 B1**
(45) Date of publication and mention of the grant of the patent: **15.04.2026**
(21) Application number: 22705945.8
(22) Date of filing: 03.02.2022
(51) Int. Cl.: A61F 5/455, A61F 5/453, A61B 10/00

(54) **EXTERNAL CATHETER WITH URINALYSIS CAPABILITIES AND RELATED SYSTEMS AND METHODS BACKGROUND**
EXTERNER KATHETER MIT HARNANALYSEFÄHIGKEITEN SOWIE ZUGEHÖRIGE SYSTEME UND VERFAHREN
CATHÉTER EXTERNE AYANT DES CAPACITÉS D'ANALYSE URINAIRE ET SYSTÈMES ET PROCÉDÉS ASSOCIÉS

(43) Date of publication of application: 11.12.2024
(73) Proprietor: Purewick Corporation, El Cajon, California 92020 (US)
(72) Inventor: ROTHBERG, Matthew Jordan, Atlanta, Georgia 30306 (US)
(74) Representative: Hoffmann Eitle
(86) International application number: PCT/US2022/015026
(87) International publication number: WO 2023/149877

(56) References cited:
- WO-A1-2019/150385
- WO-A1-2021/102296
- GB-A- 2 469 496
- US-A- 5 605 161
- US-A1- 2002 193 760
- US-A1- 2016 374 848

## Description

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

US 5,605,161 discloses a disposable urinary device comprising a collapsible funnel-shaped body to receive urine from a user in an upright position. After urination, test strips secured to the inner surface of the body can be removed from the body for urinalysis.

Bed pans and urinary catheters, such as a Foley catheter, may be used to address some of these circumstances. However, bedpans and urinary catheters have several problems associated therewith. For example, bedpans may be prone to discomfort, spills, and other hygiene issues. Urinary catheters be may be uncomfortable, painful, and may cause urinary tract infections. Conventional fluid collection devices also may be limited to use when a patient is confined to a bed in a supine position.

Thus, users and manufacturers of fluid collection devices continue to seek new and improved devices, systems, and methods to collect fluid.

### SUMMARY

US-A1-2016/0374848 discloses the features of the pre-characterizing part of claim 1 below. The invention is defined by independent claims 1 and 10. The dependent claims are directed to optional features and preferred embodiments. Embodiments disclosed herein are related to urine collection systems, urinalysis, and methods of using urine collection systems. In an embodiment, a urine collection assembly includes a fluid impermeable barrier defining at least a chamber, at least one opening, and a fluid outlet. The urine collection assembly further includes a conduit having a first end disposed in the chamber, at least one porous material disposed in the chamber, and a urinalysis device disposed in the fluid collection assembly.

In an embodiment, a urine collection system may include the urine collection assembly and a urine collection container including an interior region in fluid communication with the conduit, and a pump in fluid communication with the urine collection container and the urine collection assembly. The pump may be configured to draw the urine form the urine collection assembly and deposit the urine in the urine collection container via the conduit.

In an embodiment, a method to collect a urinalysis sample is disclosed. The method includes positioning the urine collection assembly adj acent to a urethral opening of a user. The method includes receiving urine from the urethral opening into the chamber of the urine collection assembly, saturating the urinalysis device with urine, and withdrawing the urinalysis device from within the urine collection assembly.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present invention, wherein identical reference numerals refer to identical or similar elements or features in different views or embodiments shown in the drawings.
**FIG. 1A** is a block diagram of a portable fluid collection system.
**FIG. 1B** is an isometric view of a fluid collection system.
**FIG. 2A** is a cross sectional view of a female urine collection assembly, according to an embodiment.
**FIG. 2B** is a cross sectional view of a female urine collection assembly, according to an embodiment.
**FIG. 3** is an exploded view of a female urine collection assembly, according to an embodiment.
**FIG. 4A** is a cross sectional view of a male urine collection assembly, according to an embodiment.
**FIG. 4B** is an isometric view of a male urine collection assembly, according to an embodiment.
**FIG. 4C** is a cross sectional view of the male urine collection assembly of FIG. 4B.
**FIG. 5** is a flow diagram of a method to collect a urinalysis sample, according to an embodiment.

### DETAILED DESCRIPTION

Embodiments disclosed herein are related to urine collection systems and related methods. Many users of fluid collection devices are over 65 years old with limited mobility, often relying on wheelchairs as a primary mode of transportation. Many users also have medical conditions that may require a urinalysis sample to either test, treat, or diagnose the condition. Users and caregivers, then, are benefited from a urine collection system that may be capable of providing a urinalysis sample without significantly disassembling the system, allowing users and/or caregivers to use the urine collection system to collect fluid as well as conveniently take a urinalysis sample.

In many embodiments described herein, a urine collection system may be configured to be capable of easily sampling and analyzing the urine. The urinalysis test would not require sampling from a storage container or any disassembly of the system which may lead to leaks or spills. Embodiments of the urine collection systems described herein are both able to collect urine drawn from a urine collection assembly adjacent to a urethral opening of a user and conveniently draw a urinalysis sample from the urine collection assembly.

The urine collection assembly includes a fluid impermeable barrier and an aperture in the fluid impermeable barrier. The urinalysis device may be disposed in the fluid collection assembly and be withdrawn through the aperture for sampling. In some embodiments, the urinalysis device may be configured to analyze at least one of calcium, blood, glucose, bilirubin, urobilinogen, ketones, leukocytes, creatinine, microalbumin, pH, ascorbic acid, and protein, as required by the user or as directed by a caregiver.

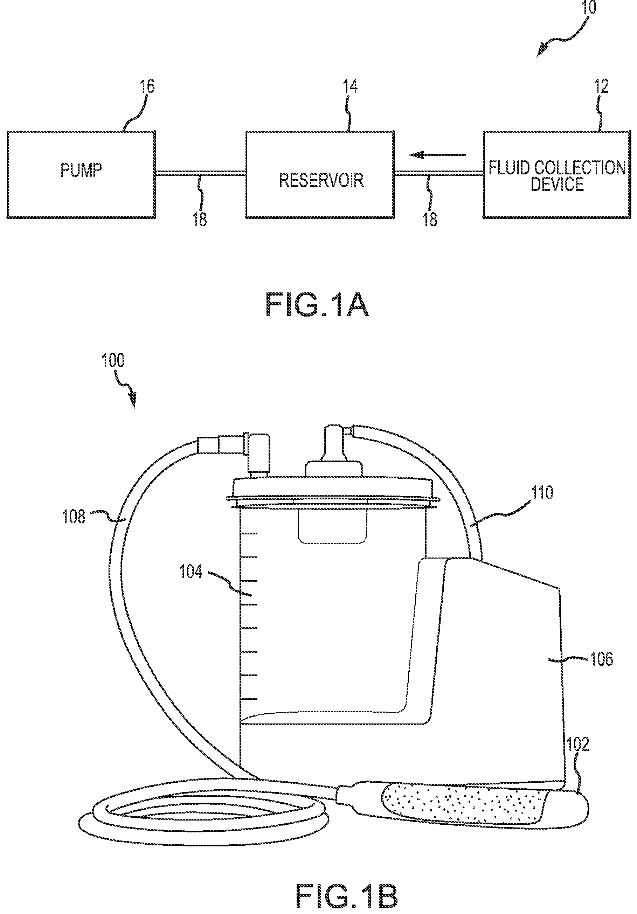

**FIG. 1A** is a block diagram of a urine collection system 10. The urine collection system 10 may be included in embodiments of fluid collection systems described herein. The system 10 includes a fluid *(e.g.,* urine) collection device 12 *(e.g.,* any of the urine collection assemblies disclosed herein), a urine collection container 14 (or reservoir), and a pump 16 (or vacuum device). The urine collection assembly 12, the urine collection container 14, and the pump 16 may be fluidly coupled to each other via one or more tubes 18. For example, urine collection assembly 10 may be operably coupled to one or more of the urine collection container 14 or the pump 16 via the tube 18. In some embodiments, the pump 16 may be coupled directly to the urine collection container 14. Fluid (*e.g*., urine or other bodily fluids) collected in the urine collection assembly 12 may be removed from the urine collection assembly 12 via the tube 18 coupled to the urine collection assembly 12. Suction force may be introduced into a chamber of the urine collection assembly 12 via the inlet of the tube 18 responsive to suction (*e.g*., vacuum) force applied at the outlet of the tube 18.

The suction force may be applied to the outlet of the tube 18 by the pump 16 either directly or indirectly. The suction force may be applied indirectly via the urine collection container 14. For example, the outlet of the tube 18 may be disposed within or fluidly coupled to an interior region of the urine collection container 14 and an additional tube 18 may extend from the urine collection container 14 to the pump 16. Accordingly, the pump 16 may apply suction to the urine collection assembly 12 via the urine collection container 14. The suction force may be applied directly via the pump 16. For example, the outlet of the tube 18 may be disposed within the pump 16. An additional tube 18 may extend from the pump 16 to a point outside of the urine collection assembly 12, such as to the urine collection container 14. In such examples, the pump 16 may be disposed between the urine collection device 12 and the urine collection container 14.

The urine collection container 14 may be sized and shaped to retain a fluid therein. The urine collection container 14 may include a bag (*e.g*., drainage bag), a bottle or cup *(e.g.,* collection jar), or any other enclosed container for storing bodily fluid(s) such as urine. In some examples, the tube 18 may extend from the urine collection assembly 12 and attach to the urine collection container 14 at a first point therein. An additional tube 18 may attach to the urine collection container 14 at a second point thereon and may extend and attach to the pump 16. Accordingly, a vacuum (*e.g*., suction) may be drawn through urine collection assembly 12 via the urine collection container 14. Fluid, such as urine, may be drained from the urine collection assembly 12 using the pump 16.

The pump 16 or vacuum source may include one or more of a manual vacuum pump, and electric vacuum pump, a diaphragm pump, a centrifugal pump, a displacement pump, a magnetically driven pump, a peristaltic pump, or any pump configured to produce a vacuum. The pump 16 may provide a vacuum or suction to remove fluid from the urine collection assembly 12. In some examples, the pump 16 may be powered by one or more of a power cord *(e.g.,* connected to a power socket), an alternator, one or more batteries, or even manual power (e.g., a hand operated vacuum pump). The vacuum sources disclosed herein may include one or more of a switch, a button, a plug, a remote, or any other device suitable to activate the pump 16.

Many embodiments of urine collection systems described herein are configured to be worn by a user, positioned on a surface such as a table, and/or securable or mountable to a wheelchair. **FIG. 1B** is an isometric view of a urine collection system 100, according to some embodiments. The urine collection system 100 includes a urine collection assembly 102 configured to receive fluid (e.g. urine) from a user. The urine collection assembly 102 may be removably attached to a urine collection container 104 of the urine collection system 100. The urine collection container 104 may be integrated into a housing 106 that may also include a pump therein. The urine collection system 100 may further include a collection tube 108 and a suction tube 110. The collection tube 108 may be disposed between the urine collection assembly 102 and the urine collection container 104. The collection tube 108 may fluidly connect the internal volume of the urine collection container 104 with the urine collection assembly 102. The suction tube 110 may couple the pump suction and/or discharge to the urine collection container 104. The pump disposed within the housing 106 may be in fluid communication with the internal volume of the urine collection container 104 via the suction tube 110. The housing 106 may also be configured to store or house internal electrical and mechanical components. For example, the housing may include a pump (not shown in **FIG. 1B**) configured to modulate pressure within the container 104.

The urine collection assembly 102 may be positioned at least proximate to a urethral opening. The urine collection assembly shown in **FIGS. 1B-2B** are examples of female fluid collection assemblies configured to collect fluid(s) from females (*e.g*., collect urine from a female urethra). Further examples of female fluid collection assemblies are disclosed in U.S. Patent No. 10,390,989 issued on August 27, 2019. However, the fluid collection assemblies, systems, and methods disclosed herein may include male fluid collection assemblies and/or devices shaped, sized, and otherwise configured to collect fluid(s) from males *(e.g.,* collect urine from a male urethra). Examples of male fluid collection assemblies are disclosed in U.S. Provisional Patent Applications No. 63/067,542 filed on August 19, 2020 and U.S. Patent Application No. 16/433,773 filed on June 6, 2019.

In some embodiments, the collection tube 108, the suction tube 110 and/or other tubing of the urine collection system 100 may include a flexible material such as materials tubing (*e.g*., medical tubing). Such material tubing may include a thermoplastic elastomer, polyvinyl chloride, ethylene vinyl acetate, polytetrafluoroethylene, flexible metal, ceramic and composite material tubing etc. The collection tube 108 may include silicon or latex. In some embodiments, the collection tube 108 may be constructed of any suitable material to be impermeable to fluids such that fluids may be drawn from the urine collection assembly 102 and into the collection tube 108. In some embodiments, the collection tube 108 may include one or more portions that are resilient, such as by having one or more of a diameter or wall thickness that allows the collection tube 108 to be flexible.

The urine collection container 104 may be integrated into the housing 106. In some embodiments, the container 104 may include at least an inlet coupled to the collection tube 108, an outlet coupled to a drain tube and a vacuum port coupled to the pump. In some embodiments, the container 104 may be opaque or clear according to different embodiments and may include a rectangular front or rear profile as shown in **FIG. 1B****.**

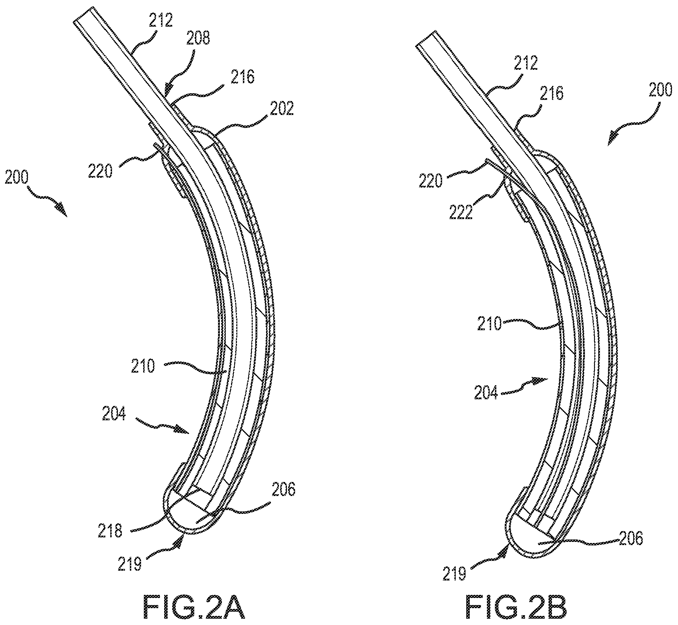

**FIG. 2A** is a cross sectional view of a urine collection assembly 200, according to an embodiment. The urine collection assembly 200 includes a fluid impermeable barrier 202, at least one opening 204, a chamber 206, a fluid outlet 208, and a porous material 210 disposed in the chamber 206 within the fluid impermeable barrier 202. A tube 212 may be at least partially disposed within the chamber 206. The inner surfaces of the fluid impermeable barrier 202 at least partially defines the chamber 206 within the fluid collection assembly 200. The fluid impermeable barrier 202 temporarily stores the bodily fluids in the chamber 206. The fluid impermeable barrier 202 may be formed of any suitable fluid impermeable material(s), such as a fluid impermeable polymer (e.g., silicone rubber, thermoplastic polyurethane, polyolefins, polyvinyl chloride etc.), a metal film, natural latex rubber, another suitable material, or combinations thereof. As such, the fluid impermeable barrier 202 substantially prevents the bodily fluids from passing through the fluid impermeable barrier 202. In some examples, the fluid impermeable barrier 202 may be tubular (ignoring the opening), such as substantially cylindrical (as shown), oblong, prismatic, or flattened tubes. During use, the fluid impermeable barrier 202 may contact the wearer. The fluid impermeable barrier 202 may be sized and shaped to fit in the gluteal cleft between the legs of a female user.

The opening 204 provides an ingress route for fluids to enter the chamber 206. The opening 204 may be defined by the fluid impermeable barrier 202 such as by an inner edge of the fluid impermeable barrier 202. For example, the opening 204 may be formed in and extend through the fluid impermeable barrier 202, thereby enabling fluid(s) to enter the chamber 206 from outside of the urine collection assembly 200. The opening 204 may be an elongated hole in the fluid impermeable barrier 202. For example, the opening 204 may be defined as a cut-out in the fluid impermeable barrier 202. The opening 204 may be located and shaped to be positioned adjacent to a female urethra.

The urine collection assembly 200 may be positioned proximate to the female urethra and bodily fluid may enter the chamber of the fluid collection assembly 200 via the opening 204. The fluid collection assembly 200 is configured to receive the fluid(s) into the chamber 206 via the opening 204. When in use, the opening 204 may have an elongated shape that extends from a first location below the urethral opening *(e.g.,* at or near the anus or the vaginal opening) to a second location above the urethral opening *(e.g.,* at or near the top of the vaginal opening or the pubic hair).

The opening 204 may have an elongated shape because the space between the legs of a female is relatively small when the legs of the female are closed, thereby only permitting the flow of the fluid(s) along a path that corresponds to the elongated shape of the opening 204 (*e.g*., longitudinally extending opening). The opening 204 in the fluid impermeable barrier 202 may exhibit a length that is measured along the longitudinal axis of the urine collection assembly 200 that may be at least about 10% of the length of the urine collection assembly 200, such as about 25% to about 50%, about 40% to about 60%, about 50% to about 75%, about 65% to about 85%, or about 75% to about 95% of the length of the urine collection assembly 200. In some embodiments, the opening 204 may be vertically oriented (*e.g*., having a major axis parallel to the longitudinal axis of the assembly 200). In other non-limiting examples (not shown), the opening 204 may be horizontally oriented (*e.g*., having a major axis perpendicular to the longitudinal axis of the assembly 200). In some embodiments, the fluid impermeable barrier 202 may be configured to be attached to the individual, such as adhesively attached (*e.g*., with a hydrogel, medical grade silicone or acrylic adhesive) to the individual. An example suitable adhesive may be a hydrogel layer.

The urine collection assembly 200 includes at least one porous material 210 disposed in the chamber 206. The porous material 210 may cover at least a portion (*e.g.,* all) of the opening 204. The porous material 210 may be exposed to the environment outside of the chamber 206 through the opening 204. The porous material 210 is configured to wick and/or allow flow of any fluid away from the opening 204, thereby preventing the fluid from escaping the chamber 206. The permeable properties referred to herein may be wicking, capillary action, diffusion, or other similar properties or processes, and are referred to herein as "permeable" and/or "wicking." Such "wicking" and "permeable" properties may not include absorption of fluid into the porous material 210. Put another way, substantially no absorption or solubility of the bodily fluids into the material may take place after the material is exposed to the bodily fluids and removed from the bodily fluids for a time. While no absorption or solubility is desired, the term "substantially no absorption" may allow for nominal amounts of absorption and/or solubility of the bodily fluids into the porous material 210 (*e.g*., absorbency), such as less than about 30 wt.% of the dry weight of the porous material 210, less than about 20 wt.%, less than about 10 wt.%, less than about 7 wt.%, less than about 5 wt.%, less than about 3 wt.%, less than about 2 wt.%, less than about 1 wt.%, or less than about 0.5 wt.% of the dry weight of the porous material 210.

In an embodiment, the porous material 210 may include at least one absorbent or adsorbent material. The porous material 210 disposed within the chamber 206 may include any material that may wick and/or allow flow of the fluid. For example, the porous material 210 may be formed from fibers from nylon *(e.g.,* spun nylon fibers), polyester, polyethylene, polypropylene, wool, silk, linen, cotton *(e.g.,* cotton gauze), felt, other fabrics and porous polymers, hydrophobic foam, an open cell foam polyurethane,, a coated porous material (*e.g*., hydrophobic coated porous material, materials with affinity to specific substances), polymeric sintered particles from polyethylene, polypropylene, polytetrafluoroethylene(PTFE), elastomeric particles, any other suitable porous materials, or combinations thereof. For example, the porous material 210 may include a body of spun nylon fibers with an outer fabric gauze layers or other permeable membrane that wraps around the body of spun nylon fibers. Forming the porous material 210 from gauze, soft fabric, and/or smooth fabric may reduce chaffing caused by the urine collection assembly 200. In some embodiments, the porous material 210 may at least substantially and/or completely fill the portions of the chamber 206 that may not be occupied by the tube 212.

The tube 212 may be at least partially disposed in the chamber 206. The tube 212 may be used to remove fluid form the chamber 206. The tube 212 may be in fluid communication with the fluid outlet 208 of the fluid collection assembly 200. The tube 212 may include a tube inlet 218 disposed in the chamber 206 and a tube outlet (not shown) positioned downstream from the tube inlet 218. The tube outlet may be operably coupled to a urine collection container (e.g. urine collection container 104. Thus, the tube 212 may fluidly couple the chamber 206 with the urine collection container (as shown in **FIGS. 1A** or **1B**).

The fluid impermeable barrier 202 defines a first aperture 216 sized appropriately to receive the tube 212. The tube 212 may be disposed in the chamber 206 via the first aperture 216. The first aperture 216 may be sized and shaped to form an at least substantially fluid tight seal against the tube 212 thereby substantially preventing the fluid(s) from escaping the chamber 206 via the first aperture 216. The first aperture may include fluid outlet 208. As shown in **FIG. 2A****,** the inlet 218 of the tube 212 may extend through the first aperture 216 and into the chamber 206. In the illustrated embodiment, the tube 212 is at least partially disposed in the chamber 206. The fluid collected in the fluid collection assembly 200 may be removed from the chamber 206 via the tube 212.

The urine collection assembly 200 may include a reservoir 219 disposed in the chamber 206. The reservoir 219 may be a substantially unoccupied portion of the chamber 206. The reservoir 219 may be at least partially defined between the fluid impermeable barrier 202 and one or both of the porous material 210 disposed in the chamber 206 and the tube inlet 218. The fluids that are in the chamber 206 may flow through the porous material 210 to the reservoir 219. The reservoir 219 may retain of the fluid therein. In some embodiments, the reservoir 219 may retain the fluids temporarily, until the fluid in the reservoir 219 is removed into the tube 212. While depicted in the end region of **FIG. 2A****,** the reservoir 219 may be located in any portion of the chamber 206. The reservoir 219 may be located in a portion of the chamber 206 that is designed to be located in a gravimetrically low point of the fluid collection device when the device is worn.

In some embodiments, the inlet 218 of the tube 212 may be located in the reservoir 219 of the urine collection assembly 200 as illustrated. In other embodiments, the inlet 218 may be aft of the reservoir 219 or flush with the porous material 210. Generally, the inlet 218 of the tube 212 may be configured within the urine collection assembly 200 such that extraction of the fluid is effective. Locating the inlet 218 of the tube 212 at or near a location expected to be the gravimetrically low point of the chamber 206 when worn by a user enables the tube 212 to receive more of the fluid(s) than if inlet 218 was located elsewhere and reduces the likelihood of pooling *(e.g.,* pooling of the fluid(s) may cause microbe growth and foul odors).

The fluid collection assembly 200 includes a urinalysis device 220 disposed within the urine collection assembly 200. In some embodiments, the urinalysis device 220 may be a basic diagnostic tool used to determine pathological changes in the urine of the user. In some embodiments, a standard urine test strip may be included. In some embodiments, the urinalysis device 220 may include a test strip. The standard urine test strip may include about 10 different chemical pads or reagents which react (change color) when immersed in, and then removed from, a urine sample. The test can often be read in as little as 60 to 120 seconds after dipping, although certain tests require longer. Routine testing of the urine with multiparameter strips is the first step in the diagnosis of a wide range of diseases. The analysis includes testing for the presence of one or more proteins, glucose, ketones, haemoglobin, bilirubin, urobilinogen, acetone, nitrite or leucocytes as well as testing of pH and specific gravity or to test for infection by different pathogens.

In some embodiments, the test strips can include a ribbon made of plastic or paper of about 5 millimeter wide. Plastic strips may include pads impregnated with chemicals that react with the compounds present in urine producing a characteristic color. For the paper strips the reactants may be absorbed directly onto the paper. Paper strips may be specific to a single reaction (e.g. pH measurement), while the strips with pads may conduct several determinations simultaneously.

There are strips which serve different purposes, such as qualitative strips that only determine if the sample is positive or negative, or there are semi-quantitative ones that in addition to providing a positive or negative reaction also provide an estimation of a quantitative result, in the latter the color reactions are approximately proportional to the concentration of the substance being tested for in the sample. The reading of the results is carried out by comparing the pad colors with a color scale provided by the manufacturer, no additional equipment is needed.

In some embodiments, the urinalysis device 220 may be configured to extend longitudinally across the at least one opening 204. The urinalysis device 220 may be configured to include a width that is finite and less than the width of the at least one opening 204 such that the urinalysis device 220 does not obstruct the opening 204 and/or reduce the ingress of urine through the opening 204 and into the chamber 206. In some embodiments, the urinalysis device 220 may be disposed within the chamber 206. In some embodiments, the urinalysis device 220 may be disposed within the at least one porous material 210. In other embodiments, the urinalysis device 220 may be disposed in front of the porous material 210, in reference to the at least one opening 204.

**FIG. 2B** is a cross sectional view of a urine collection assembly, according to an embodiment. The fluid impermeable barrier 202 includes a second aperture 222 sized appropriately to receive the urinalysis device 220. The urinalysis device 220 may be disposed in the chamber 206 via the second aperture 222. The second aperture 222 may be sized and shaped to form an at least substantially fluid tight seal against the urinalysis device 220 thereby substantially preventing the fluid(s) from escaping the chamber 206 via the second aperture 222. As shown in **FIG. 2B****,** the urinalysis device 220 may extend through the second aperture 222 and into the chamber 206. The urinalysis device 220 may extend across the at least one opening 204. In some embodiments, the second aperture 222 may be located proximate to the fluid outlet 208. In the illustrated embodiment, the urinalysis device 220 is at least partially disposed in the chamber 206.

In some embodiments, the urinalysis device 220 may be disposed within the tube 212. In some embodiments the tube 212 may provide a convenient location for a caretaker to remove and analyze the device 220. In some embodiments, the tube 212 may include an aperture wherein the urinalysis device 220 may be withdrawn and/or inserted to be saturated.

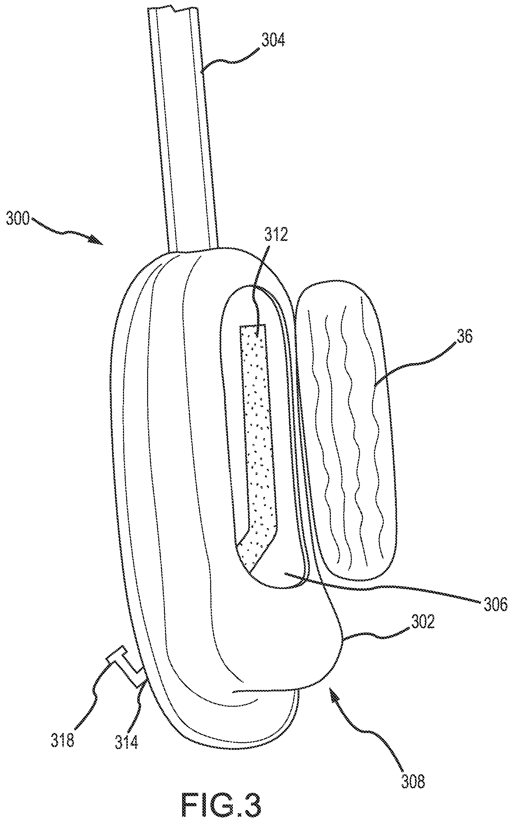

**FIG. 3** is an exploded view of another example urine collection assembly 300, according to an embodiment. The urine collection assembly 300 includes a fluid impermeable barrier 302 shaped to be configured to be positioned at least proximate to a urethra of a user. The urine collection assembly 300 includes a conduit 304 in fluid communication with the urine collection assembly 300 and configured to couple to a urine collection container (not shown). The urine collection assembly 300 also includes an opening 306. Urine may pass through the opening 306 and into a chamber 308 disposed within the urine collection assembly 300. The chamber 308 includes a porous material 310. In some embodiments, a urinalysis device 312 can be disposed within the urine collection assembly 300. In some embodiments, the urinalysis device 312 may be disposed within the conduit 304. The fluid impermeable barrier 302 includes an aperture 314. The urinalysis device 312 is configured to be withdrawn through the aperture 314. The urinalysis device 312 can be configured to extend longitudinally across the opening 306 of the urine collection assembly 300.

The aperture 314 is disposed on an exterior surface 316 of the fluid impermeable barrier 302. The aperture 314 may be disposed in an area convenient to access for the user and/or caretaker. In some embodiments, the urinalysis device 312 can include a pull tab 318. The pull tab 318 can include a portion of the urinalysis device 312 that extends past the fluid impermeable barrier 302 to provide a portion of the device that can be gripped to remove the urinalysis device 312 from the urine collection assembly 300. In some embodiments, the pull tab 318 can be configured to prevent the urinalysis device 312 from slipping through the aperture 314 and being completely within the chamber and/or within the fluid impermeable barrier 302. In some embodiments, the pull tab 318 may include a textured surface to enhance the grip of the user or caretaker. In some embodiments, the urinalysis device 312 may be configured to analyze at least one of calcium, bold, glucose, bilirubin, urobilinogen, ketonses, leukocytes, creatinine, microalbumin, pH, ascorbic acid, and/or protein.

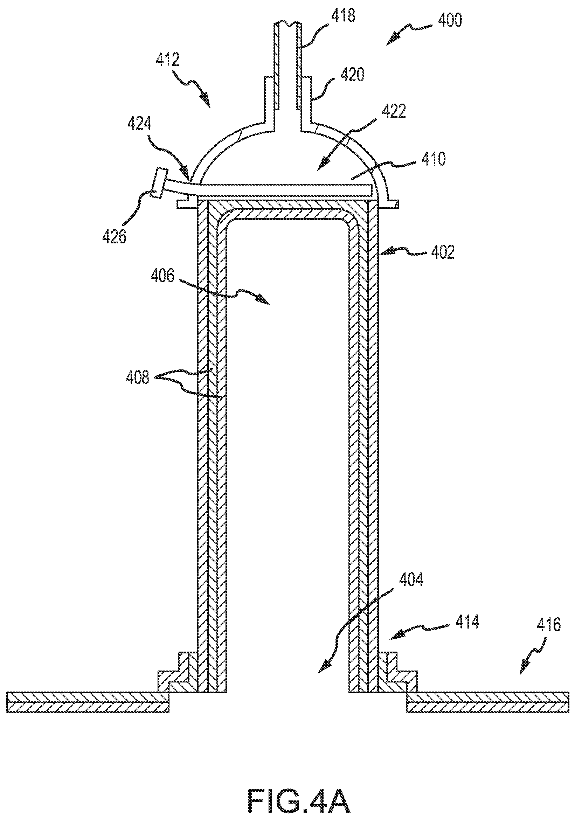

**FIG. 4A** is a cross sectional view of a male urine collection assembly 400, according to an embodiment. In other words, the urine collection assembly 400 is designed to collect fluid(s) from male users. The urine collection assembly 400 includes a fluid impermeable barrier 402 shaped to be configured to be positioned around a penis of the user. The fluid impermeable barrier 402 may be tubular or cup-shaped. The fluid impermeable barrier 402 defines an opening 404 extending through the fluid impermeable barrier 402 that is configured to have at least a portion of a penis positioned therethrough.

The fluid impermeable barrier 402 at least partially defines a chamber 406 within the urine collection assembly 400. The fluid impermeable barrier 402 temporarily stores the fluid(s) in the chamber 406. The fluid impermeable barrier 402 may be formed of any suitable fluid impermeable material(s) to substantially prevent the urine from passing through the fluid impermeable barrier 402. The fluid impermeable barrier 402 at least partially defines the chamber 406. For example, an inner surface of the fluid impermeable barrier 402 at least partially defines the perimeter of the chamber 406.

The urine collection assembly 400 includes at least one porous material 408. The porous material 408 is configured to wick any fluid away from the opening 404, thereby preventing the fluid from escaping the chamber 406. The porous material 408 may include any material that may wick the fluid. For example, the porous material 408 may include fabric, such as a gauze *(e.g.,* a silk, linen, or cotton gauze), another soft fabric, or another smooth fabric. Forming the porous material 408 from gauze, soft fabric, and/or smooth fabric may reduce chaffing caused by the urine collection assembly 400. The porous material 408 may not completely fill the chamber 406 since the chamber 406 is configured to have an unoccupied space that receives at least a portion of the penis. The urine collection assembly 400 may include more than one layer of porous material 408.

The urine collection assembly 400 may include a reservoir 410. The reservoir 410 may be a substantially unoccupied portion of the chamber 406. The fluid(s) that are in the chamber 406 may flow through the porous material 408 to the reservoir 410. The reservoir 410 may retain the fluid(s) therein. In some embodiments, the reservoir 410 may be located at a distal end 412 of the urine collection assembly 400 and the opening may be at a proximal end 414 of the urine collection assembly. The proximal end 414 may include a base 416 that may be sized, shaped, and made of a material to be coupled to skin that surrounds the penis and have at least the urethral opening of the penis positioned therethrough. The base 416 may include the opening 404. In some embodiments, the base 416 may include a rigid structure configured to maintain the fluid impermeable membrane 402 in a particular position and/or at a particular angle relative to the individuals body (*e.g.,* the skin about the penis).

The fluid impermeable barrier 402 also includes at least a portion of a tube 418 disposed therein, such as at least partially disposed in the reservoir 410. In an example, the tube 418 may extend from a first aperture 420 at the distal region 412 to the proximal region 414 at least proximate to the opening 404. The proximal region 414 may be disposed near or on the skin around the penis *(e.g.,* on the penis or pubic area therearound). Accordingly, when a patient lays on their back, fluid *(e.g.,* urine) may aggregate near the opening 404 of the fluid impermeable barrier 402 against the skin of the subject. The fluid may be removed from the chamber 406 and/or the reservoir 410 via the tube 418. The first aperture 420 may be sized and shaped to form an at least substantially fluid tight seal against the tube 418 thereby substantially preventing the fluid(s) from escaping the chamber 406 via the first aperture 420.

The urine collection assembly 400 further includes a urinalysis device 422. In some embodiments, the urinalysis device 422 may include a test strip. In some embodiments, the urinalysis device 422 may be configured to extend across the chamber 406 and/or the reservoir 410. The urinalysis device 422 may be configured to include a width that is finite and less than the width of the chamber 406 such that the urinalysis device 422 does not obstruct the flow of urine into the tube 418 and/or reduce the ingress of urine through the opening 404 and into the chamber 406. In some embodiments, the urinalysis device 422 may be disposed within the chamber 406. In some embodiments, the urinalysis device 422 may be disposed within the at least one porous material 408. In other embodiments, the urinalysis device 422 may be disposed in the distal end 412 of the urine collection assembly 400.

The fluid impermeable barrier 402 includes a second aperture 424. The urinalysis device 422 can be configured to be withdrawn through the second aperture 424. The urinalysis device 422 can be configured to extend across the reservoir 410, distal to the porous material 408 and proximal to the tube 418.

In some embodiments, the second aperture 424 can be disposed on an exterior surface of the fluid impermeable barrier 402. The second aperture 424 may be disposed in an area convenient to access for the user and/or caretaker. In some embodiments, the urinalysis device 422 may include a pull tab 426. The pull tab 426 can include a portion of the urinalysis device 422 that extends past the fluid impermeable barrier 402 to provide a portion of the device that can be gripped to remove the urinalysis device 422 from the urine collection assembly 400. In some embodiments, the pull tab 424 can be configured to prevent the urinalysis device 422 from slipping through the second aperture 424 and being completely within the chamber and/or within the fluid impermeable barrier 402.

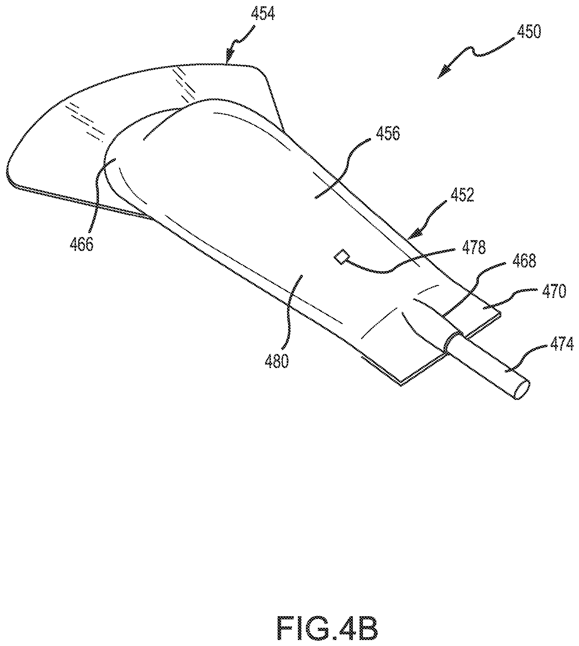

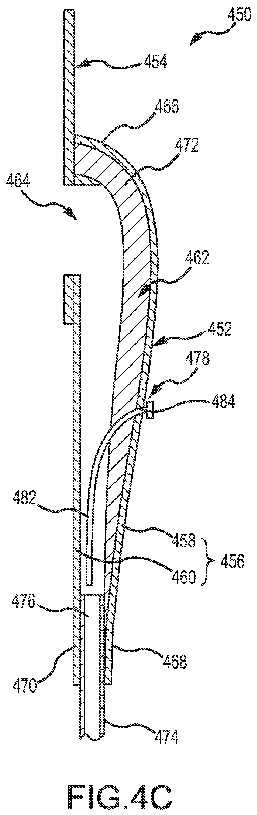

**FIG. 4B** and **FIG. 4C** are an isometric view and a cross sectional view, respectively, of a male urine collection assembly 450, according to an embodiment. The urine collection assembly 450 includes a sheath 452 and a base 454. The sheath 452 includes a fluid impermeable barrier 456 that is at least partially formed from a first panel 458 attached to a second panel 460. However, in other embodiments, the first panel 458 and the second panel 460 may be integrally formed with each other. The fluid impermeable barrier 456 also defines a chamber 462 between the first panel 458 and the second panel 460, an opening 464 at a proximal end region 466 of the sheath 452 and the base 454, and an outlet 468 at a distal end region 470 of the sheath 452. The sheath 452 also includes at least one porous material 472 disposed in the chamber 112. The base 454 is permanently attached to the proximal end region 466 of the sheath 452.

The porous material 472 is disposed within the chamber and between the first panel 458 and the second panel 460. The opening 464 is formed in and extends through the fluid impermeable barrier 456, thereby enabling bodily fluids to enter the chamber 462 from outside of the urine collection assembly 450. The opening 464 may be configured to receive a penis of the user and the penis can extend into the chamber 462.

The fluid impermeable barrier 456 defines the outlet 468 sized to receive a tube 474. The tube 474 may be at least partially disposed in the chamber 462 or otherwise in fluid communication with the chamber 462 through the outlet 468. The outlet 468 may be sized and shaped to form an at least substantially fluid tight seal against the tube 474 thereby substantially preventing the bodily fluids from escaping the chamber 462. An inlet 476 of the tube 474 may be located at or near the distal end region 470 of the sheath 452 which is expected to be the gravimetrically low point of the chamber 462 when worn by a user. Locating the inlet 476 at or near the distal end region 470 of the sheath 452 enables the tube 474 to receive more of the bodily fluids than if the inlet 476 was located elsewhere and reduce the likelihood of pooling (*e.g*., pooling of the bodily fluids may cause microbe growth and foul odors). For instance, the bodily fluids in porous material 472 due to capillary forces. However, the bodily fluids may exhibit a preference to flow in the direction of gravity, especially when at least a portion of the porous material 472 is saturated with the bodily fluids. Accordingly, the inlet 476 may be located in the fluid collection assembly 450 in a position expected to be the gravimetrically low point in the urine collection assembly 450 when worn by a user.

The urine collection assembly 450 includes an aperture 478 in an outer surface 480 of the fluid impermeable barrier 456. The urine collection assembly 450 may further include a urinalysis device 482. In some embodiments, the urinalysis device 482 may include a test strip. The urinalysis device 482 may be configured to extend across the chamber 462 and towards the distal end region 470 of the sheath 452. The urinalysis device 482 may be configured to include a width that is finite and less than the width of the chamber 462 such that the urinalysis device 482 does not obstruct the flow of urine into the tube 474 and/or reduce the ingress of urine through the opening 464 and into the chamber 462. The urinalysis device 482 may be disposed within the chamber 462. In some embodiments, the urinalysis device 462 may be disposed within the at least one porous material 472.

The urinalysis device 482 is configured to be withdrawn through the aperture 478 in the outer surface 480 of the fluid impermeable barrier 456. Similar to the previously disclosed assemblies, the aperture 478 may be disposed in an area convenient to access for the user and/or caretaker. In some embodiments, the urinalysis device 482 may include a pull tab 484. The pull tab 484 can include a portion of the urinalysis device 482 that extends past the outer surface 480 of the fluid impermeable barrier 456 to provide a portion of the device that can be gripped to remove the urinalysis device 482 from the urine collection assembly 450. In some embodiments, the pull tab 484 can be configured to prevent the urinalysis device 482 from slipping through the aperture 478 and being completely within the chamber 462 and/or within the fluid impermeable barrier 456.

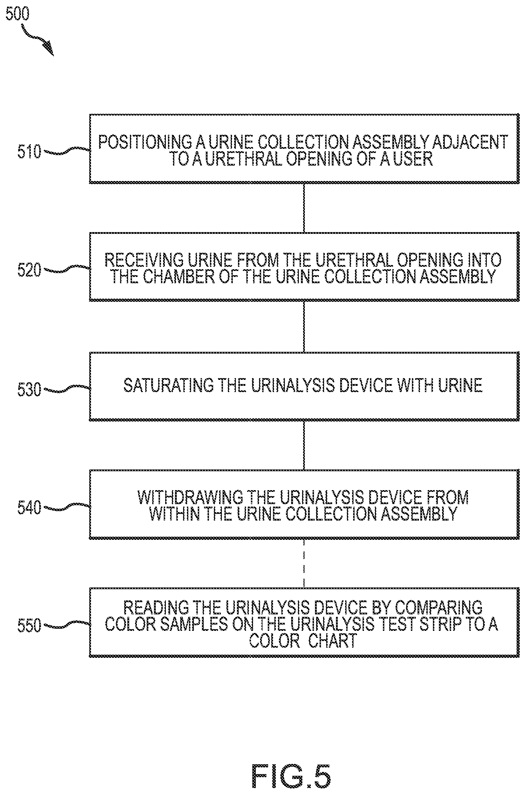

**FIG. 5** is a flow diagram of a method 500 to collect a urinalysis sample, according to an embodiment. The method 500 includes an act 510 of positioning a urine collection assembly adjacent to a urethral opening of a user. The urine collection assembly includes a fluid impermeable barrier defining at least a chamber, at least one opening, and a fluid outlet. The urine collection assembly further includes at least one porous material disposed in the chamber, and a urinalysis device disposed in the chamber. In some embodiments, the urinalysis device may be disposed within the at least one porous material. The method 500 can further include an act 520 of receiving urine from the urethral opening into the chamber of the urine collection assembly. The method 500 also includes an act 530 of saturating the urinalysis device with urine. The method 500 may also include an act 540 of withdrawing the urinalysis device from within the urine collection assembly. In some embodiments, the urinalysis device may be withdrawn immediately after the urinalysis device is saturated. In other embodiments, the urinalysis device may be withdrawn prior to replacing and disposing of the urine collection assembly. In some embodiments, withdrawing the urinalysis device may include withdrawing the test strip through an aperture in the fluid impermeable barrier. The urinalysis device may generally be withdrawn manually by the user or the caretaker. Generally, the urinalysis device is withdrawn and read manually or placed in a machine/computer for further analysis.

In some embodiments, the method 500 may further include an act 550 of reading the urinalysis test strip by comparing color samples on the urinalysis test strip to a color chart. In some embodiments, the urinalysis device may be read by an automated urinalysis analyzer. The method 500 may further include applying suction with a pump effective to suction the urine from the chamber via a conduit and depositing the urine in a urine collection container. The urine collection container may include either a reusable container or a disposable container.

The acts of the method of collecting fluids from a user described above are for illustrative purposes. For example, the acts of the method of collecting fluids from a user and collecting a urinalysis sample can be performed in different orders, split into multiple acts, modified, supplemented, or combined. In an embodiment, one or more of the acts of the method of collecting fluids from a user can be omitted from the method. Any of the acts of the method of collecting a urinalysis sample from a user can include using any of the urine collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

## Claims

1. A urine collection assembly (200), comprising:
a fluid impermeable barrier (202) defining at least a chamber (206), at least one opening (204), and a fluid outlet (208) provided by a first aperture;
a conduit extending through the first aperture and including a first end disposed in the chamber the conduit being for removing urine from the chamber by suction;
at least one porous material (210) disposed in the chamber to wick urine away from the opening; and **characterized in that**:
a urinalysis device (220) is disposed in the chamber, and **in that**:
the fluid impermeable barrier includes a second aperture (222) and the urinalysis device is configured to be withdrawn through the second aperture.

2. The urine collection assembly of claim 1, wherein the second aperture is located proximate to the fluid outlet.

3. The urine collection assembly of any of the previous claims, wherein the urinalysis device extends across the at least one opening.

4. The urine collection assembly of any of claims 1-3, wherein the urinalysis device is disposed within the at least one porous material.

5. The urine collection assembly of any of claims 1-3, wherein the urinalysis device is disposed within the conduit.

6. The urine collection assembly of any of the previous claims, wherein the urinalysis device is configured to analyze at least one of calcium, blood, glucose, bilirubin, urobilinogen, ketones, leukocytes, creatinine, microalbumin, pH, ascorbic acid, and protein.

7. The urine collection assembly of any of the previous claims, wherein the urinalysis device includes a urinalysis test strip.

8. The urine collection assembly of any of the previous claims, wherein the urinalysis device includes a pull tab (318).

9. A urine collection system, comprising:
a urine collection assembly as claimed in any of the preceding claims, together with
a pump (18) in fluid communication with the urine collection container and the urine collection assembly, the pump configured to draw the urine from the urine collection assembly and deposit the urine in the urine collection container via the conduit.

10. A method to collect a urinalysis sample, the method comprising:
i) positioning, adjacent to a urethral opening, a urine collection assembly as claimed in any of claims 1 to 8;
ii) receiving urine from the urethral opening into the chamber of the urine collection assembly;
iii) saturating the urinalysis device with urine; and
iv) withdrawing the urinalysis device from within the chamber of the urine collection assembly, through the second aperture.

11. The method of claim 10, wherein the urinalysis device is disposed within the at least one porous material.

## Patentansprüche

1. Urinsammelanordnung (200), umfassend:
eine fluidundurchlässige Barriere (202), die mindestens eine Kammer (206), mindestens eine Öffnung (204) und einen Fluidauslass (208) definiert, der durch eine erste Öffnung bereitgestellt wird;
eine Leitung, die sich durch die erste Öffnung erstreckt und ein erstes Ende einschließt, das in der Kammer angeordnet ist, wobei die Leitung dazu dient, Urin durch Ansaugen aus der Kammer zu entfernen;
mindestens ein poröses Material (210), das in der Kammer angeordnet ist, um Urin von der Öffnung wegzuleiten; und **dadurch gekennzeichnet, dass**:
eine Urinanalysevorrichtung (220) in der Kammer angeordnet ist, und dass:
die fluidundurchlässige Barriere eine zweite Öffnung (222) einschließt und die Urinanalysevorrichtung so konfiguriert ist, dass sie durch die zweite Öffnung herausgezogen werden kann.

2. Urinsammelanordnung nach Anspruch 1, wobei sich die zweite Öffnung in der Nähe des Fluidauslasses befindet.

3. Urinsammelanordnung nach einem der vorstehenden Ansprüche, wobei sich die Urinanalysevorrichtung über die mindestens eine Öffnung erstreckt.

4. Urinsammelanordnung nach einem der Ansprüche 1 - 3, wobei die Urinanalysevorrichtung innerhalb des mindestens einen porösen Materials angeordnet ist.

5. Urinsammelanordnung nach einem der Ansprüche 1 - 3, wobei die Urinanalysevorrichtung innerhalb der Leitung angeordnet ist.

6. Urinsammelanordnung nach einem der vorstehenden Ansprüche, wobei die Urinanalysevorrichtung so konfiguriert ist, dass sie mindestens einen der folgenden Werte analysiert: Calcium, Blut, Glukose, Bilirubin, Urobilinogen, Ketone, Leukozyten, Kreatinin, Mikroalbumin, pH-Wert, Ascorbinsäure und Protein.

7. Urinsammelanordnung nach einem der vorstehenden Ansprüche, wobei die Urinanalysevorrichtung einen Urinanalyse-Teststreifen einschließt.

8. Urinsammelanordnung nach einem der vorstehenden Ansprüche, wobei die Urinanalysevorrichtung eine Zuglasche (318) einschließt.

9. Urinsammelsystem, umfassend:
eine Urinsammelanordnung nach einem der vorstehenden Ansprüche, zusammen mit einer Pumpe (18), die in Fluidverbindung mit dem Urinsammelbehälter und der Urinsammelanordnung steht, wobei die Pumpe konfiguriert ist, dass sie den Urin aus der Urinsammelanordnung ansaugt und den Urin über die Leitung in den Urinsammelbehälter abscheidet.

10. Verfahren zum Sammeln einer Urinanalyseprobe, wobei das Verfahren Folgendes umfasst:
i) Positionieren einer Urinsammelanordnung gemäß einem der Ansprüche 1 bis 8 benachbart an einer Harnröhrenöffnung;
ii) Aufnehmen von Urin aus der Harnröhrenöffnung in die Kammer der Urinsammelanordnung;
iii) Sättigen der Urinanalysevorrichtung mit Urin; und
iv) Zurückziehen der Urinanalysevorrichtung durch die zweite Öffnung aus der Kammer der Urinsammelanordnung.

11. Verfahren nach Anspruch 10, wobei die Urinanalysevorrichtung innerhalb des mindestens einen porösen Materials angeordnet ist.

## Revendications

1. Ensemble de recueil d'urine (200), comprenant :
une barrière imperméable aux fluides (202) définissant au moins une chambre (206), au moins une ouverture (204), et une sortie de fluide (208) définie par un premier orifice ;
un conduit s'étendant à travers le premier orifice et incluant une première extrémité disposée dans la chambre, le conduit servant à retirer l'urine de la chambre par aspiration ;
au moins un matériau poreux (210) disposé dans la chambre pour assurer un effet de mèche et acheminer l'urine à distance de l'ouverture ; et **caractérisé en ce que** :
un dispositif d'analyse urinaire (220) est disposé dans la chambre, et **en ce que** :
la barrière imperméable aux fluides inclut un deuxième orifice (222) et le dispositif d'analyse urinaire est configuré pour être retiré à travers le deuxième orifice.

2. Ensemble de recueil d'urine selon la revendication 1, dans lequel le deuxième orifice est situé à proximité de l'orifice de sortie de fluide.

3. Ensemble de recueil d'urine selon l'une quelconque des revendications précédentes, dans lequel le dispositif d'analyse urinaire s'étend en travers de ladite au moins une ouverture.

4. Ensemble de recueil d'urine selon l'une quelconque des revendications 1 à 3, dans lequel le dispositif d'analyse urinaire est disposé à l'intérieur dudit au moins un matériau poreux.

5. Ensemble de recueil d'urine selon l'une quelconque des revendications 1 à 3, dans lequel le dispositif d'analyse urinaire est disposé à l'intérieur du conduit.

6. Ensemble de recueil d'urine selon l'une quelconque des revendications précédentes, dans lequel le dispositif d'analyse urinaire est configuré pour analyser au moins un de calcium, sang, glucose, bilirubine, urobilinogène, cétones, leucocytes, créatinine, microalbumine, pH, acide ascorbique, et protéine.

7. Ensemble de recueil d'urine selon l'une quelconque des revendications précédentes, dans lequel le dispositif d'analyse urinaire inclut une bandelette de test d'analyse urinaire.

8. Ensemble de recueil d'urine selon l'une quelconque des revendications précédentes, dans lequel le dispositif d'analyse urinaire inclut une languette de traction (318).

9. Système de recueil d'urine, comprenant :
un ensemble de recueil d'urine selon l'une quelconque des revendications précédentes, ainsi qu'une pompe (18) en communication fluidique avec le récipient de recueil d'urine et l'ensemble de recueil d'urine, la pompe étant configurée pour aspirer l'urine depuis l'ensemble de recueil d'urine et déposer l'urine dans le récipient de recueil d'urine via le conduit.

10. Procédé pour recueillir un échantillon d'analyse urinaire, le procédé comprenant :
i) positionner, adjacent à une ouverture urétrale, un ensemble de recueil d'urine selon l'une quelconque des revendications 1 à 8 ;
ii) recueillir l'urine provenant de l'ouverture urétrale dans la chambre de l'ensemble de recueil d'urine ;
iii) saturer le dispositif d'analyse urinaire avec de l'urine ; et
iv) retirer le dispositif d'analyse urinaire de l'intérieur de la chambre de l'ensemble de recueil d'urine, à travers le deuxième orifice.

11. Procédé selon la revendication 10, dans lequel le dispositif d'analyse urinaire est disposé à l'intérieur dudit au moins un matériau poreux.
